# EUROPEAN PATENT SPECIFICATION

(11) **EP 3 907 278 B1**
(45) Date of publication and mention of the grant of the patent: **02.07.2025**
(21) Application number: 20904261.3
(22) Date of filing: 22.12.2020
(51) Int. Cl.: C12N 5/071

(54) **METHOD FOR MANUFACTURING CELL SUSPENSION AND METHOD FOR MANUFACTURING ADHERENT CELL**
VERFAHREN ZUR HERSTELLUNG EINER ZELLSUSPENSION UND VERFAHREN ZUR HERSTELLUNG EINER ADHÄRENTEN ZELLE
PROCÉDÉ DE FABRICATION D'UNE SUSPENSION CELLULAIRE ET PROCÉDÉ DE FABRICATION D'UNE CELLULE ADHÉRENTE

(30) Priority: 13.03.2020 JP 2020043974
(43) Date of publication of application: 10.11.2021
(73) Proprietor: Minaris Regenerative Medicine Co., Ltd., Yokohama, Kanagawa 221-0024 (JP)
(72) Inventor: CHEN, Shangwu, Tokyo 1006606 (JP); NAKAGAWA, Fumiko, Tokyo 1006606 (JP); TADA, Yasuhiko, Tokyo 1008280 (JP); OKANOJO, Masahiro, Tokyo 1006606 (JP); SATO, Yushi, Tokyo 1006606 (JP); SUZUKI, Yuma, Tokyo 1006606 (JP); NAKASHIMA, Katsuhiko, Tokyo 1006606 (JP); TAKAHASHI, Ryosuke, Tokyo 1006606 (JP)
(74) Representative: Winter, Brandl - Partnerschaft mbB
(86) International application number: PCT/JP2020/047981
(87) International publication number: WO 2021/181819

(56) References cited:
- JP-A- 2008 515 412
- YANG JIANJUN ET AL: "Large-scale microcarrier culture of HEK293T cells and Vero cells in single-use bioreactors", AMB EXPRESS, 24 May 2019 (2019-05-24), Berlin/Heidelberg, pages 1 - 14, XP055877466, Retrieved from the Internet <URL:https://amb-express.springeropen.com/track/pdf/10.1186/s13568-019-0794-5.pdf> [retrieved on 20220111], DOI: 10.1186/s13568-019-0794-5
- SCHIRMAIER CARMEN ET AL: "Scale-up of adipose tissue-derived mesenchymal stem cell production in stirred single-use bioreactors under low-serum conditions", ENGINEERING IN LIFE SCIENCES, vol. 100 ml, 2l, 35l14, no. 3, 1 May 2014 (2014-05-01), DE, pages 292 - 303, XP055877699, ISSN: 1618-0240, DOI: 10.1002/elsc.201300134
- JOSSEN VALENTIN ET AL: "Manufacturing human mesenchymal stem cells at clinical scale: process and regulatory challenges", APPLIED MICROBIOLOGY AND BIOTECHNOLOGY, vol. 102, no. 9, 1 May 2018 (2018-05-01), Berlin/Heidelberg, pages 3981 - 3994, XP055877692, ISSN: 0175-7598, Retrieved from the Internet <URL:https://link.springer.com/content/pdf/10.1007/s00253-018-8912-x.pdf> DOI: 10.1007/s00253-018-8912-x
- SWARTZ ELLIOT: "SBE Special Section: Industrial Biotechnology", 1 October 2019 (2019-10-01), XP055877709, Retrieved from the Internet <URL:https://gfi.org/wp-content/uploads/2021/01/Cell-Based_Meat_CEP_Oct2019-2.pdf> [retrieved on 20220111]
- CHEN SHANGWU ET AL: "Facile bead-to-bead cell-transfer method for serial subculture and large-scale expansion of human mesenchymal stem cells in bioreactors", STEM CELLS TRANSLATIONAL MEDICINE, vol. 10, no. 9, 18 May 2021 (2021-05-18), US, pages 1329 - 1342, XP055877871, ISSN: 2157-6564, Retrieved from the Internet <URL:https://onlinelibrary.wiley.com/doi/full-xml/10.1002/sctm.20-0501> DOI: 10.1002/sctm.20-0501
- CHIN, S. B.: "P02-113: 50L large scale culture of mesenchymal stem cells for regenerative medicine", REGENERATIVE MEDICINE, vol. 19, 12 March 2020 (2020-03-12) - 14 March 2020 (2020-03-14), JP, pages 795, XP009531401, ISSN: 1347-7919
- ADLERZ K., TREMPEL M., WANG D., KIRIAN R.D., ROWLEY J.A., AHSAN T.: "Comparison of msc-evs manufatured in 2D versus scalable 3D bioreactor systems", CYTOTHERAPY, ISIS MEDICAL MEDIA, OXFORD,, GB, vol. 21, no. 5, 1 May 2019 (2019-05-01), GB , pages S58, XP055859057, ISSN: 1465-3249, DOI: 10.1016/j.jcyt.2019.03.434
- DE SOURE, A. M. ET AL.: "Scalable microcarrier- based manufacturing of mesenchymal stem/stromal cells", JOURNAL OF BIOTECHNOLOGY, vol. 236, 2016, pages 88 - 109, XP029729470, DOI: 10.1016/j.jbiotec.2016.08.007
- CHEN, A. K. L. ET AL.: "Application of human mesenchymal and pluripotent stem cell microcarrier cultures in cellular therapy: Achievements and future direction", BIOTECHNOLOGY ADVANCES, vol. 31, 2013, pages 1032 - 1046, XP055113967, DOI: 10.1016/j.biotechadv.2013.03.006
- LAWSON, T. ET AL.: "Process development for expansion of human mesenchymal stromal cells in a 50 L single-use stirred tank bioreactor", BIOCHEMICAL ENGINEERING JOURNAL, vol. 120, 2017, pages 49 - 62, XP029932798, DOI: 10.1016/j.bej.2016.11.020
- RAFIQ, Q. A. ET AL.: "Qualitative and quantitative demonstration of bead-to-bead transfer with bone marrow-derived human mesenchymal stem cells on microcarriers: Utilising the phenomenon to improve culture performance", BIOCHEMICAL ENGINEERING JOURNAL, vol. 135, 2018, pages 11 - 21, XP085400608, DOI: 10.1016/j.bej.2017.11.005

## Description

### TECHNICAL FIELD

The present disclosure relates to a method for manufacturing a cell suspension, a method for manuractureing adherent cells, a method for manuracturing a useful substance-containing liquid, and a method for manufacturing a useful substance.

### BACKGROUND ART

Adherent cells are cells which require a scaffold for proliferation. Generally, the culture of adherent cells uses a culture carrier that functions as the scaffold. In those cases where these types of adherent cells are to be used in cell preparations or useful substance production or the like, large-scale expansion of the adherent cells is necessary. Biochemical Engineering Journal, 120 (2017), pp. 49 to 62 examines a method for large-scale culture of human mesenchymal stromal cells using microcarriers as culture carriers.

On the other hand, extracellular vesicles such as exosomes are known as substances that are secreted from cells. WO 2009 / 105 044 examines a particle manufacturing method that includes isolating a particle containing at least one biological property of a mesenchymal stem cell from a mesenchymal stem cell conditioned medium (MSC-CM).

### SUMMARY OF INVENTION

### PROBLEMS INVENTION AIMS TO SOLVE

Stem cells have a proliferation ability and a differentiation potential, and therefore offer much potential for clinical applications to regenerative medicine. However, clinical applications require extremely large numbers of stem cells. Further, exosomes isolated from stem cells contain a variety of biologically active substances, and therefore have potential for use in disease treatment methods and diagnostic methods. Obtaining an adequate amount of an exosome also requires a large number of stem cells. However, there is currently still considerable room for improvement in terms of techniques for the large-scale culture of stem cells.

Accordingly, the present disclosure provides a method for efficient large-scale manufacturing of cell suspensions. Further, the present disclosure also provides a method for efficient large-scale manufacturing of adherent cells. Moreover, the present disclosure also provides a method for efficient large-scale manufacturing of useful substance-containing liquids, and a method for efficient large-scale manufacturing of useful substances.

### MEANS FOR SOLUTION OF THE PROBLEMS

This object is solved by the subject matter of the independent claims. Further aspects are disclosed in the subclaims. This description includes various embodiments of the present invention. Examples of these embodiments are presented below.

### EFFECTS OF THE INVENTION

The present disclosure provides a method for efficient large-scale manutacturing of cell suspensions. Further, the present disclosure also provides a method for efficient large-scale manufacturing of adherent cells. Moreover, the present disclosure also provides a method for efficient large-scale manufacturing of useful substance-containing liquids, and a method for efficient large-scale manufacturing of useful substances.

### BRIEF DESCRIPTION OF THE DRAWINGS

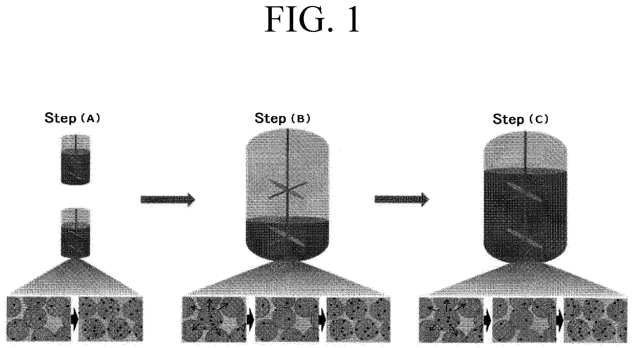

FIG. 1 is schematic diagram illustrating a method for manufacturing a cell suspension in the examples.

### EMBODIMENTS FOR CARRYING OUT THE INVENTION

Embodiments of the present invention are described below. The present invention is not limited to the following embodiments. The following embodiments can be implemented individually or in combination.

### <Method for Manufacturing Cell Suspension>

### [Manufacturing Method Including Steps (A), (B) and (C)]

In one embodiment of the present disclosure, a method for manufacturing a cell suspension includes (A), (B) and (C) described below:
(A) culturing adherent cells in a cell suspension containing the adherent cells, a microcarrier and a medium, and having a volume of at least 0.3 L,
(B) culturing the adherent cells in a cell suspension containing the adherent cells obtained through (A), a microcarrier and a medium, and having a volume of at least 5 L, and
(C) culturing the adherent cells in a cell suspension containing the adherent cells obtained through (B), a microcarrier and a medium, and having a volume of at least 10 L.

In this description, (A), (B) and (C) described above are sometimes referred to as step (A), step (B) and step (C) respectively. However, the term "step" includes not only independent steps, but also steps which are not clearly distinguishable from other steps, provided that the operation defined in the step is executed, even if the step is not clearly distinguishable.

By using the method for manufacturing a cell suspension that includes the steps (A), (B) and (C), large-scale culture of adherent cells can be achieved easily, and following the large-scale culture, a uniform cell population can be obtained at a favorable survival rate. In the large-scale culture of adherent cells, a method is sometimes used in which the adherent cells are detached from a culture carrier that functions as a scaffold and then subcultured. However, the passage operations accompanying the separation of the adherent cells and the microcarriers via detachment or the like can sometimes cause contamination or damage or the like of the adherent cells. In the present disclosure, for the cell suspension for culturing containing a fresh microcarrier, by using a simple method in which the steps (A), (B) and (C) are conducted in sequence while specific volume control is undertaken, the migration of the adherent cells between microcarriers can be promoted and an efficient subculturing of the adherent cells can be achieved, enabling large-scale culture of the adherent cells to be realized.

The term "cell suspension" means a liquid that contains cells, and these cells may exist in a state adhered to microcarriers or in an unadhered state.

### [Adherent Cells]

There are no particular limitations on the adherent cells, provided they are known to exhibit adhesion to the selected substrate, and examples include somatic cells and stem cells and the like. Examples of the somatic cells include endothelial cells, epidermal cells, epithelial cells, myocardial cells, myoblasts, nerve cells, bone cells, osteoblasts, fibroblasts, adipose cells, hepatic cells, renal cells, pancreatic cells, adrenal gland cells, periodontal ligament cells, gingival cells, periosteal cells, skin cells, dendritic cells and macrophages.

The adherent cells are preferably animal-derived cells, and are more preferably mammalianderived cells. Examples of the mammals include humans, monkeys, chimpanzees, cows, pigs, horses, sheep, goats, rabbits, rats, mice, marmots, dogs and cats. The adherent cells may be cells derived from the skin, liver, kidneys, muscles, bone, blood vessels, blood, or tissue such as nerve tissue. A single type of cell is typically cultured independently, but a combination of two or more types of cell may also be cultured. The cells may be primary cells from tissue, or may be a cell line established by immortalization. Moreover, the cells may also be artificially established cells.

In one embodiment, the adherent cells may be stem cells. Examples of the stem cells include somatic stem cells such as mesenchymal stem cells, hematopoietic stem cells, neural stem cells, myeloid stem cells and germline stem cells, and either mesenchymal stem cells or myeloid mesenchymal stem cells may be used. The term "mesenchymal stem cells" refers broadly to somatic stem cells which exist in various human tissues, and can be differentiated into all or some mesenchymal cells such as osteoblasts, chondrocytes and adipocytes. Stem cells may also include induced pluripotent stem cells (iPS cells) and embryonic stem cells (ES cells). In one embodiment, the method for manufacturing a cell suspension according to the present disclosure is a method that is suitable for large-scale manufacturing of mesenchymal stem cells.

### [Microcarrier]

The microcarrier is a carrier that functions as the scaffold for cell proliferation during culturing of the adherent cells. Microcarriers that are known as carriers for cell culture may be used. The material for the microcarrier may be an organic substance, an inorganic substance or a composite material thereof, and may be either dissolvable or indissolvable. Examples of the organic substances include synthetic polymers such as polystyrene, polyester, polyurethane, polyethylene, polypropylene, polyvinyl alcohol, (meth)acrylic-based polymers, (meth)acrylamide-based polymers, silicone-based polymers, epoxy resins and urethane resins; and natural polymers such as cellulose, dextran, collagen, polygalacturonic acid, polyalginic acid and gelatin. Examples of the inorganic substances include glass, ceramics, metals, alloys and metal oxides. From the viewpoint of cytocompatibility, the material for the microcarrier preferably contains an organic substance, and more preferably contains a natural polymer. Form the viewpoint of operability, a dissolvable microcarrier is preferred, but the microcarrier is not limited to such microcarrier. In this description, the term "dissolvable microcarrier" means a microcarrier that can be dissolved using an enzyme or the like to the point where the adhered cells can be released from the microcarrier.

From the viewpoint of promoting cell adhesion, cationic functional groups may be introduced onto the surface of the microcarrier. Examples of the cationic functional groups include groups containing substituted or unsubstituted amino groups such as dimethylamino groups, diethylamino groups and amino groups. Further, from the viewpoint of promoting cell adhesion, a cell-adhesive polymer may be disposed at the surface of the microcarrier. The cell-adhesive polymer may be a polypeptide or polysaccharide that exhibits cell adhesion, and examples include collagen, gelatin, alginic acid, Matrigel^{™} (manufactured by BD Biosciences Ltd.), hyaluronic acid, laminin, fibronectin, vitronectin, elastin, heparan sulfate, dextran, dextran sulfate and chondroitin sulfate. The cell-adhesive polymer may also be a partial peptide or oligosaccharide that exhibits cell adhesion.

Examples of the shape of the microcarrier include spherical shapes, flat shapes, cylindrical shapes, plate shapes and prismatic shapes. The microcarrier preferably includes a spherical microcarrier. The microcarrier may be a porous microcarrier having internal pores or a microcarrier that has no internal pores.

From the viewpoint of promoting cell proliferation, the average particle size (D50) of the microcarrier is, for example, within a range from 50 to 1,000 µm, and is preferably from 100 to 500 µm, and more preferably from 150 to 250 µm. The average particle size of the microcarrier is the value measured for the median size (D50) in a physiological saline solution or in a medium. The average particle size of the microcarrier can be measured using a laser diffraction/scattering-type particle size distribution analyzer.

A fresh microcarrier is used as the microcarrier. In this description, a "fresh microcarrier" means a microcarrier that has not been used as a carrier (scaffold) for cell culture, namely, an unused microcarrier. In this description, a "used microcarrier" means a microcarrier that has already been used as a carrier for cell culture.

The concentration of the microcarrier in the suspension may be adjusted appropriately depending on the shape, size, and surface area and the like of the microcarrier, but for example, may be set within a range from 0.01 to 100 g/L, from 0.5 to 50 g/L, or from 1 to 20 g/L.

### [Medium]

In the manufacturing methods according to the present disclosure, a liquid medium is used as the medium. The medium preferably contains an inorganic salt, an amino acid, sugar and water. The medium may also contain optional components such as blood serum, nucleosides and/or nucleotides, vitamins, hormones, antibiotics, growth factors and adhesion factors. Media that are known as basal media for cell culture may be used as the medium.

Any medium that is known for use in culturing the selected cells may be used as the medium without any particular limitations, and examples include DMEM (Dulbecco's Modified Eagle's Medium), MEM (Eagle's Minimum Essential Medium), αMEM medium (α-Modified Eagle's Minimum Essential Medium), GMEM (Glasgow Minimum Essential Medium), IMDM (Iscove's Modified Dulbecco's Medium), Ham's F12 (Ham's Nutrient Mixture F12), RPMI-1640 (RPMI-1640 medium), McCoy's 5A (McCoy's 5A Medium), MSC growth medium 2 (manufactured by PromoCell GmbH), Prime XV XSFM (manufactured by Irvine Scientific, Inc.), and mixtures of two or more media selected from these media. Conventional media other than the above media can also be used, and in particular, media that are known for use in the culture of stem cells may be used. The medium used for culturing may be free of xenogeneic components. Media that are free of xenogeneic components may include serum substitute additives (for example, Knockout Serum Replacement (KSR) (manufactured by Invitrogen Corporation), Chemically-defined Lipid Concentrate (manufactured by Gibco Inc.), and Glutamax (manufactured by Gibco Inc.) and the like) instead of animal-derived serum.

From the viewpoint of promoting cell proliferation, the medium preferably contains a nucleoside and/or nucleotide, and more preferably contains a nucleoside. The nucleoside may be a ribonucleoside, a deoxyribonucleoside, or a mixture thereof. The nucleotide may be a ribonucleotide, a deoxyribonucleotide, or a mixture thereof. Examples of the bases included in these nucleosides and nucleotides include purine bases such as adenine and guanine, and pyrimidine bases such as cytosine, thymine and uracil. In those cases where the medium contains a nucleoside and/or nucleotide, the concentration in the medium may be set within a range from 1 to 20 mg/L, or from 5 to 10 mg/L.

### [Culture]

The conditions for culturing the adherent cells may be adjusted in accordance with the cell variety to achieve conditions suitable for cell proliferation. The culture temperature may be set, for example, within a range from 20 to 45°C, and is preferably from 30 to 40°C. The carbon dioxide concentration may be set, for example, within a range from 1 to 20% by volume, and is preferably from 3 to 15% by volume. **In** the case of mammalian cells, a temperature of 37°C and a carbon dioxide concentration of 5% (v/v) are generally used. Examples of the culture vessel include a flask, a bioreactor, a tank or a culture bag.

Culture may be conducted by agitating (stirring) or shaking the cell suspension. Each step may also include a period where the agitation or shaking is halted. The agitation is only intermittent agitation, or a combination of intermittent agitation and continuous agitation. The descriptions, configurations, examples and conditions and the like for the intermittent agitation may each be applied, independently, to steps (A), (B) and/or (C).

The culture is preferably conducted by agitating or shaking the cell suspension in the culture vessel with the microcarrier floating in the cell suspension. The agitation method may be selected appropriately in accordance with the type and size of the culture vessel, which is selected in accordance with the volume of the cell suspension described below, and examples include methods that use a magnetic stirrer, mechanical stirrer, homo mixer, homogenizer, or vortex mixer or the like. Examples of the shaking method include methods that use a shaker. In one embodiment, from the viewpoint of obtaining a suspension having the microcarrier dispersed favorably within the cell suspension, agitation of the cell suspension is preferred.

The agitation rate at which the microcarrier can be dispersed favorably in the cell suspension is dependent on the shape and volume of the culture vessel, but generally, in the case of a stirred tank bioreactor with a volume of 1 to 50 L, the agitation rate may be set within a range from 30 to 200 rpm, and is preferably from 40 to 100 rpm. The agitation rate during culturing may be altered in accordance with the floating state of the microcarrier or cells.

### [Step (A)]

In step (A), the adherent cells are cultured in a cell suspension containing the adherent cells, a microcarrier and a medium, and having a volume of at least 0.3 L. As a result of the culturing, the adherent cells adhere to the microcarrier and proliferate, yielding a cell suspension containing adherent cells adhered to the microcarrier. In other words, the adherent cells obtained through step (A) include a population of adherent cells that are adhered to the microcarrier. Culturing may be conducted for a prescribed time period. From the viewpoint of enabling large-scale culture of the adherent cells in a uniform and efficient manner, the volume of the cell suspension is, for example, at least 0.3 L, at least 0.5 L, or 1 L or greater. Although there are no particular limitations on the upper limit for the volume of the cell suspension, from the viewpoints of efficiency and economic viability, the volume may be set, for example, to not more than 10 L, or 5 L or less.

In step (A), a cell suspension containing the adherent cells, a fresh microcarrier and a medium, and having a volume of at least 0.3 L is obtained, and the adherent cells are cultured. In this description, the cell suspension obtained in step (A) prior to culturing is sometimes referred to as "the cell suspension (A1)". Further, in this description, the cell suspension obtained in step (A) following culturing is sometimes referred to as "the cell suspension (A2)".

The cell suspension (A1) can be obtained, for example, by mixing at least the adherent cells, a fresh microcarrier, and a medium. More specifically, the cell suspension (A1) can be obtained by mixing, at least, adherent cells that are not adhered to the microcarrier, a fresh microcarrier, and a fresh medium. From the viewpoint of enabling large-scale culture of the adherent cells in a uniform and efficient manner, the volume of the cell suspension (A1) is, for example, at least 0.3 L, at least 0.5 L, or 1 L or greater. Although there are no particular limitations on the upper limit for the volume of the cell suspension (A1), from the viewpoints of efficiency and economic viability, the volume may be set, for example, to not more than 10 L, or 5 L or less.

The concentration of the adherent cells in the cell suspension (A1) is, for example, within a range from 1×10³ to 2×10⁵ cells/mL, and is preferably from 5×10³ to 1×10⁵ cells/mL, and more preferably from 1×10⁴ to 5×10⁴ cells/mL. The concentration of a fresh microcarrier in the cell suspension (A1) is, for example, within a range from 0.1 to 50 g/L, and is preferably from 0.5 to 10 g/L and more preferably from 1 to 5 g/L.

The culture period in step (A) differs depending on the cell seeding density, the cell type, and the culturing conditions and the like, but generally, the period may be set to yield a state where satisfactory cell growth has occurred, for example, a period which, based on the cell adhesive region for the microcarrier (the region for which adhesion of the adherent cells is possible), yields at least 80% confluence, at least 90% confluence, at least 95% confluence, or 100% confluence. In the present disclosure, the cell growth state in the cell adhesive region can be observed with a fluorescence microscope (manufactured by Keyence Corporation). Specifically, by determining the percentage of the expanded surface area of the cells adhered to the microcarrier surface relative to the surface area of the microcarrier, the growth state of the cells can be confirmed. The culture period may be, for example, a period of 2 to 14 days.

In step (A), from the viewpoint of efficiency, the culturing of the adherent cells may be conducted by agitating the cell suspension (A1) under appropriate culturing conditions in which the temperature and carbon dioxide concentration and the like have been adjusted. The agitation is only intermittent agitation, or a combination of intermittent agitation and continuous agitation. In one embodiment, step (A) includes conducting intermittent agitation of the cell suspension (A1), and then conducting continuous agitation of the cell suspension obtained through the intermittent agitation. The intermittent agitation and continuous agitation are described below in further detail. Intermittent agitation may be conducted after the continuous agitation, or a combination of intermittent agitation and continuous agitation may be conducted repeatedly. In another embodiment, the agitation in step (A) involves only intermittent agitation of the cell suspension (A1).

The volume of the cell suspension (A2) obtained in step (A) may be at least 0.3 L, at least 0.5 L, or 1 L or greater. In step (A), medium may be added to the cell suspension (A1) during culturing, and/or a portion or all of the medium contained within the cell suspension (A1) may be replaced with a fresh medium during culturing. During step (A), two or more cell suspensions (A1) undergoing culturing may also be combined. In some cases, adherent cells not adhered to a microcarrier may also be added to the cell suspension (A1) during culturing. Although there are no particular limitations on the upper limit for the volume of the cell suspension (A2), from the viewpoints of efficiency and economic viability, the volume may be set, for example, to not more than 10 L, or 5 L or less.

### [Step (B)]

In step (B), the adherent cells are cultured in a cell suspension containing the adherent cells obtained through step (A), a microcarrier and a medium, and having a volume of at least 5 L. As a result of the culturing, the adherent cells adhered to the microcarrier obtained in step (A) migrate and adhere to another microcarrier, and preferably a fresh microcarrier, and undergo proliferation, thus obtaining a cell suspension containing adherent cells adhered to the microcarrier. In other words, the adherent cells obtained through step (B) include a population of adherent cells that are adhered to the microcarrier. Culturing may be conducted for a prescribed time period. The volume of the cell suspension can be set, for example, to at least 5 L, at least 8 L, or 10 L or greater. From the viewpoint of enabling efficient large-scale culture of the adherent cells, the volume of the cell suspension may be set, for example, to not more than 50 L, not more than 40 L, or 30 L or less.

In step (B), a cell suspension containing the adherent cells obtained through step (A), a fresh microcarrier and a medium, and having a volume of at least 5 L is obtained, and these adherent cells are cultured. In this description, the cell suspension obtained in step (B) prior to culturing is sometimes referred to as "the cell suspension (B 1)". Further, in this description, the cell suspension obtained in step (B) following culturing is sometimes referred to as "the cell suspension (B2)".

The cell suspension (B1) can be obtained, for example, by mixing at least the adherent cells obtained through step (A), a fresh microcarrier, and a medium. More specifically, the cell suspension (B1) can be obtained by mixing, at least, a portion or all of the cell suspension (A2), a fresh microcarrier, and a fresh medium. The mixing may also use a combination of two or more separate cell suspensions (A2) obtained by conducting independent steps (A). In this case, the cell suspension (B1) will contain adherent cells derived from the two or more cell suspensions (A2).

In step (B), the culture vessel used in step (A) and containing the cell suspension (A1) may be used, or a different culture vessel from that used in step (A) may be used. In the former case, the cell suspension (B1) can be obtained by adding a fresh microcarrier and medium to the culture vessel. In the latter case, the cell suspension (B1) can be obtained by adding the cell suspension (A1), a fresh microcarrier and the medium to the culture vessel. In either case, there are no particular limitations on the order of the addition.

From the viewpoint of enabling efficient large-scale culture of the adherent cells, the volume of the cell suspension (B1) may be, for example, at least 5 L, at least 8 L, or 10 L or greater. From the viewpoint of enabling efficient large-scale culture of the adherent cells, the volume of the cell suspension (B1) may be set, for example, to not more than 50 L, not more than 40 L, or 30 L or less. The volume of the cell suspension (B1) is greater than the volume of the cell suspension (A2). From the viewpoint of enabling an efficient scaling up from the cell suspension (A2) to the cell suspension (B1), the ratio between the volume of the cell suspension (B1) and the volume of the cell suspension (A2) ([volume of cell suspension (B1)] / [volume of cell suspension (A2)]) is, for example, within a range from 1.5 to 20, and preferably from 2 to 10, and more preferably from 3 to 6. Particularly in those cases where the cell suspension (B1) is obtained by mixing a portion or all of the cell suspension (A2), a fresh microcarrier and a fresh medium, from the viewpoint of enabling an efficient scaling up from the cell suspension (A2) to the cell suspension (B1), the ratio between the volume of the cell suspension (B1) and the total volume of the cell suspension (A2) used in the mixing ([volume of cell suspension (B1)] / [total volume of cell suspension (A2) used in mixing]) is, for example, within a range from 1.5 to 20, preferably from 2 to 10, and more preferably from 3 to 8. In this description, the term "scaling up" means increasing the volume of the culture environment.

The concentration of adherent cells in the cell suspension (B1) is, for example, within a range from 1×10³ to 2×10⁵ cells/mL, and is preferably from 5×10³ to 1×10⁵ cells/mL, and more preferably from 1×10⁴ to 5×10⁴ cells/mL. The concentration of a fresh microcarrier in the cell suspension (B1) is, for example, within a range from 0.1 to 50 g/L, and is preferably from 0.5 to 10 g/L and more preferably from 1 to 5 g/L.

The culture period in step (B) differs depending on the cell seeding density, the cell type, and the culturing conditions and the like, but generally, a period may be set to yield a state where satisfactory cell growth has occurred, for example, a period which, based on the cell adhesive region for the microcarrier, yields at least 80% confluence, at least 90% confluence, at least 95% confluence, or 100% confluence. The culture period may be, for example, a period of 2 to 10 days.

In step (B), from the viewpoint of efficiency, the culturing of the adherent cells may be conducted by agitating the cell suspension (B1) under appropriate culturing conditions in which the temperature and carbon dioxide concentration and the like have been adjusted. The agitation is only intermittent agitation, or a combination of intermittent agitation and continuous agitation. In one embodiment, step (B) includes conducting intermittent agitation of the cell suspension (B1), and then conducting continuous agitation of the cell suspension obtained through the intermittent agitation. The intermittent agitation and continuous agitation are described below in further detail. Intermittent agitation may be conducted after the continuous agitation, or a combination of intermittent agitation and continuous agitation may be conducted repeatedly. In another embodiment, the agitation in step (B) involves only intermittent agitation of the cell suspension (B1).

The volume of the cell suspension (B2) obtained in step (B) may be, for example, at least 5 L, at least 8 L, or 10 L or greater. In step (B), medium may be added to the cell suspension (B1) during culturing, and/or a portion or all of the medium contained within the cell suspension (B1) may be replaced with a fresh medium during culturing. During step (B), two or more cell suspensions (B1) undergoing culturing may also be combined. In some cases, adherent cells not adhered to a microcarrier may also be added to the cell suspension (B1) during culturing. Although there are no particular limitations on the upper limit for the volume of the cell suspension (B2), from the viewpoint of enabling efficient large-scale culture of the adherent cells, the volume may be set, for example, to not more than 50 L, not more than 40 L, or 30 L or less.

### [Step (C)]

In step (C), the adherent cells are cultured in a cell suspension containing the adherent cells obtained through step (B), a microcarrier and a medium, and having a volume of at least 10 L. As a result of the culturing, the adherent cells adhered to the microcarrier obtained in step (B) migrate and adhere to another microcarrier, and preferably a fresh microcarrier, and undergo proliferation, thus obtaining a cell suspension containing adherent cells adhered to the microcarrier. In other words, the adherent cells obtained through step (C) include a population of adherent cells that are adhered to the microcarrier. Culturing may be conducted for a prescribed time period. From the viewpoint of enabling efficient large-scale culture of the adherent cells, the volume of the cell suspension may be set, for example, to at least 10 L, at least 20 L, or 30 L or greater. From the viewpoint of enabling efficient large-scale culture of the adherent cells, the volume of the cell suspension may be set, for example, to not more than 500 L, not more than 300 L, not more than 150 L, not more than 100 L, or 80 L or less.

In step (C), a cell suspension containing the adherent cells obtained through step (B), a fresh microcarrier and a medium, and having a volume of at least 10 L is obtained, and these adherent cells are cultured. In this description, the cell suspension obtained in step (C) prior to culturing is sometimes referred to as "the cell suspension (C1)". Further, in this description, the cell suspension obtained in step (C) following culturing is sometimes referred to as "the cell suspension (C2)".

The cell suspension (C1) can be obtained, for example, by mixing at least the adherent cells obtained through step (B), a fresh microcarrier, and a medium. More specifically, the cell suspension (C1) can be obtained by mixing, at least, a portion or all of the cell suspension (B2), a fresh microcarrier, and a fresh medium. The mixing may also use a combination of two or more separate cell suspensions (B2) obtained by conducting independent steps (B). In this case, the cell suspension (C1) will contain adherent cells derived from the two or more cell suspensions (B2).

From the viewpoint of enabling efficient large-scale culture of the adherent cells, the volume of the cell suspension (C1) may be, for example, at least 10 L, at least 20 L, or 30 L or greater. From the viewpoint of enabling efficient large-scale culture of the adherent cells, the volume of the cell suspension (C1) may be set, for example, to not more than 500 L, not more than 300 L, not more than 150 L, not more than 100 L, or 80 L or less. The volume of the cell suspension (C1) is greater than the volume of the cell suspension (B2). From the viewpoint of enabling an efficient scaling up from the cell suspension (B2) to the cell suspension (C1), the ratio between the volume of the cell suspension (C1) and the volume of the cell suspension (B2) ([volume of cell suspension (C1)] / [volume of cell suspension (B2)]) is, for example, within a range from 1.5 to 20, and preferably from 2 to 10, and more preferably from 2 to 6. Particularly in those cases where the cell suspension (C1) is obtained by mixing a portion or all of the cell suspension (B2), a fresh microcarrier and a fresh medium, from the viewpoint of enabling an efficient scaling up from the cell suspension (B2) to the cell suspension (C1), the ratio between the volume of the cell suspension (C1) and the total volume of the cell suspension (B2) used in the mixing ([volume of cell suspension (C1)] / [total volume of cell suspension (B2) used in mixing]) is, for example, within a range from 1.5 to 10, preferably from 1.8 to 6, and more preferably from 2 to 3.

The concentration of adherent cells in the cell suspension (C1) is, for example, within a range from 1×10³ to 2×10⁵ cells/mL, and is preferably from 5×10³ to 1×10⁵ cells/mL, and more preferably from 1×10⁴ to 5×10⁴ cells/mL. The concentration of a fresh microcarrier in the cell suspension (C1) is, for example, within a range from 0.1 to 50 g/L, and is preferably from 0.5 to 10 g/L and more preferably from 1 to 5 g/L.

The culture period in step (C) differs depending on the cell seeding density, the cell type, and the culturing conditions and the like, but generally, the period may be set to yield a state where satisfactory cell growth has occurred, for example, a period which, based on the cell adhesive region for the microcarrier, yields at least 80% confluence, at least 90% confluence, at least 95% confluence, or 100% confluence. The culture period may be, for example, a period of 3 to 20 days.

In step (C), from the viewpoint of efficiency, the culturing of the adherent cells may be conducted by agitating the cell suspension (C1) under appropriate culturing conditions in which the temperature and carbon dioxide concentration and the like have been adjusted. The agitation is only intermittent agitation, or a combination of intermittent agitation and continuous agitation. In one embodiment, step (C) includes conducting intermittent agitation of the cell suspension (C1), and then conducting continuous agitation of the cell suspension obtained through the intermittent agitation. The intermittent agitation and continuous agitation are described below in further detail. Intermittent agitation may be conducted after the continuous agitation, or a combination of intermittent agitation and continuous agitation may be conducted repeatedly. In another embodiment, the agitation in step (C) involves only intermittent agitation of the cell suspension (C1).

The volume of the cell suspension (C2) obtained in step (C) may be at least 10 L, at least 20 L, or 30 L or greater. In step (C), medium may be added to the cell suspension (C1) during culturing, and/or a portion or all of the medium contained within the cell suspension (C1) may be replaced with a fresh medium during culturing. During step (C), two or more cell suspensions (C1) undergoing culturing may also be combined. In some cases, adherent cells not adhered to a microcarrier may also be added to the cell suspension (C1) during culturing. Although there are no particular limitations on the upper limit for the volume of the cell suspension (C2), from the viewpoint of economic viability, the volume may be set, for example, to not more than 500 L, not more than 300 L, not more than 150 L, not more than 100 L, or 80 L or less.

### [Examples of Manufacturing Methods including Steps (A), (B) and (C)]

In the present disclosure, a method for manufacturing a cell suspension includes the steps (A), (B) and (C). By implementing steps (A), (B) and (C), a cell suspension containing adherent cells (for example, the cell suspension (C2)) can be obtained. In each step, the adherent cells undergo subculturing. The method for manufacturing a cell suspension may include two or more repetitions of each step. Optional steps may also be included between each pair of steps. In one embodiment, the total number of steps represented by step (A), step (B) and step (C) within the method for manufacturing a cell suspension is preferably not more than 6 steps, more preferably not more than 5 steps, and even more preferably either 3 or 4 steps. In one embodiment, the total number of steps represented by "scaling up from the cell suspension (A2) to the cell suspension (B1)" and "scaling up from the cell suspension (B2) to the cell suspension (C1)" within the method for manufacturing a cell suspension is preferably not more than 5 steps, more preferably not more than 4 steps, and even more preferably either 2 or 3 steps. Two steps is particularly desirable.

Examples of the manufacturing method include a manufacturing method that includes one step (A), one step (B), and one step (C); a manufacturing method that includes a plurality of independent steps (A), one step (B) that uses a combination of the two or more cell suspensions (A2) obtained above, and one step (C) (a total of 4 or more steps); and a manufacturing method that includes one step (A), a first step (B), a second step (B) that uses the cell suspension (B2) obtained in the first step (B), and one step (C) (a total of 4 steps).

Examples of optional steps that may be included in the method for manufacturing a cell suspension include steps for thawing frozen cells, washing cells, seeding cells, adding microcarriers, adding a medium or at least one component contained in the medium to the cell suspension, replacing at least a portion of the medium contained in the cell suspension, leaving the cell suspension to stand, separating the cells and the microcarrier, and conducting medium replacement, cell culturing, or medium replacement and cell culturing either prior to step (A) or after step (C). In one embodiment, the method for manufacturing a cell suspension does not include a step of separating the microcarrier and the adherent cells such as a step of detaching the adherent cells from the microcarrier. In those cases where the method does not include a step of separating the microcarrier and the adherent cells, contamination and damage and the like of the adherent cells is suppressed, the functionality exhibited by the adherent cells can be favorably maintained, and the economic and timewise efficiency of the cell culture can be improved.

### [Method for Manufacturing Cell Suspension that includes Intermittent Agitation and Continuous Agitation]

In another embodiment of the present disclosure, the method for manufacturing a cell suspension includes obtaining a cell suspension containing adherent cells, a microcarrier and a medium, intermittently agitating the cell suspension, and continuously agitating the cell suspension obtained through the intermittent agitation. Examples of these cell suspensions include, but are not limited to, the cell suspension (A1), the cell suspension (B1), and the cell suspension (C1) and the like described above. The adherent cells, microcarrier and medium and the like are as described above. This method for manufacturing a cell suspension may also include one or more optional steps such as thawing frozen cells, washing cells, seeding cells, adding a medium or at least one component contained in the medium to the cell suspension, replacing at least a portion of the medium contained in the cell suspension, leaving the cell suspension to stand, and separating the cells and the microcarrier.

In the present disclosure, by using a manufacturing method that includes intermittent agitation and continuous agitation, large-scale culture of adherent cells can be achieved. Generally, in the cell culture of adherent cells using a microcarrier, continuous agitation is conducted to achieve a favorable state of dispersion of the microcarrier in the cell suspension undergoing culturing. In contrast, in the present disclosure, by using the simple method of combining intermittent agitation and continuous agitation, a favorable state of suspension for the microcarrier was able to be obtained, while large-scale culture of the adherent cells was achieved. To explain further, it is thought that the microcarrier precipitation which can sometimes occur during continuous agitation and intermittent agitation is due to variation in the microcarrier weight caused by variations in the amount of adhered adherent cells, and aggregation or the like of microcarriers to which no adherent cells are adhered. It can be surmised that this type of precipitation can be resolved by using the simple method of combining intermittent agitation and continuous agitation, thus enabling a favorable state of suspension to be achieved for the microcarrier, and enabling large-scale culture of the adherent cells to be realized. Further, in the present disclosure, in addition to using this agitation method, by also adding a fresh microcarrier to the culture system, thereby combining the so-called bead-to-bead culturing method in which the adherent cells migrate from microcarriers that have completed adhesion to other microcarriers having a larger surface area available for adhesion, resulting in more efficient proliferation, larger scale culture was able to be achieved. However, the present disclosure is not constrained by this theory.

In the present disclosure, intermittent agitation refers to conducting agitation for a prescribed period and then not conducting agitation for a prescribed period. Intermittent agitation refers to alternately conducting agitation for a prescribed period and then not conducting agitation for a prescribed period. The expression "alternately conducting" may mean repeating the combination of conducting agitation and not conducting agitation two or more times (wherein the combination of "conducting agitation" and subsequently "not conducting agitation" is counted as one repetition). The time for which agitation is conducted and the time for which agitation is not conducted may be the same or different. Further, in the case of two or more repetitions, the time for which agitation is conducted and/or the time for which agitation is not conducted may be the same or different across each repetition. The time for which agitation is not conducted may be the time for which the cell suspension is allowed to stand.

The time for which agitation is conducted is within a range from 0.5 to 60 minutes, preferably from 1 to 20 minutes, and more preferably from 3 to 10 minutes. The time for which agitation is not conducted is within a range from 0.1 to 10 hours, preferably from 0.5 to 6 hours, and more preferably from 1 to 3 hours. Examples of combinations include "agitation for 0.5 to 60 minutes followed by no agitation for 0.1 to 10 hours", but "agitation for 1 to 20 minutes followed by no agitation for 0.5 to 6 hours" is preferred, and "agitation for 3 to 10 minutes followed by no agitation for 1 to 3 hours" is more preferred.

The time for which intermittent agitation is conducted (the total time for two or more repetitions of "conducting agitation" and then "not conducting agitation") is, for example, within a range from 1 to 80 hours, preferably from 10 to 40 hours, and more preferably from 20 to 30 hours.

The continuous agitation conducted after the intermittent agitation refers to conducting agitation continuously for a prescribed period. The time period for which this agitation is conducted is, for example, within a range from 1 to 14 days, preferably from 2 to 10 days, and more preferably from 3 to 7 days. During cell culture, agitation may sometimes be halted temporarily, for example to add medium to the cell suspension or to replace the medium. Here, a temporary halt typically does not mean simply leaving the cell suspension to stand. The aforementioned time for which agitation is conducted need not be the total of the agitation time prior to a temporary halt and the agitation time after the temporary halt. In other words, the time for which agitation is conducted may be measured in segments caused by a halt in the agitation.

The intermittent agitation and continuous agitation may be conducted in a continuous manner, or a prescribed period during which no agitation is conducted may be included between the intermittent agitation and the continuous agitation. This prescribed period is, for example, within a range from 0.1 to 24 hours, preferably from 0.5 to 5 hours, and more preferably from 1 to 2 hours. This period of no agitation may involve leaving the cell suspension to stand. Following the continuous agitation, intermittent agitation may be conducted, or a combination of intermittent agitation and continuous agitation may be conducted repeatedly.

The total culture time, including the intermittent agitation and the continuous agitation, may be set appropriately in accordance with the type of adherent cells being cultured, the purpose of the culture, and the culturing conditions and the like.

### <Method for Manufacturing Adherent Cells>

In one embodiment of the present disclosure, a method for manufacturing adherent cells includes providing a cell suspension obtained using one of the embodiments described above, and obtaining the adherent cells from the cell suspension. The method for manufacturing adherent cells may also include other optional steps. The cell suspension obtained using the method for manufacturing a cell suspension contains adherent cells that are adhered to a microcarrier.

By collecting the adherent cells adhered to the microcarrier in the cell suspension using conventional separation methods such as supernatant removal and centrifugal separation, the adherent cells can be obtained in a state adhered to the microcarrier. By subsequently detaching and then harvesting the adherent cells from the microcarrier using a conventional detachment method such as an enzyme treatment, the adherent cells can be obtained. Alternatively, the adherent cells can be obtained by dissolving the microcarrier using a conventional dissolution method and then collecting the adherent cells.

In another embodiment, a prescribed enzyme or the like is added to the cell suspension containing the adherent cells adhered to the microcarrier, the adherent cells are detached from the microcarrier, or the microcarrier is dissolved, or a combination of both these techniques is used, manufacturing a state where the adherent cells can be collected in a standalone state. Subsequently, these adherent cells in a collectable state can be harvested using a conventional separation device such as a filter, thus obtaining the adherent cells.

### <Method for Manufacturing Useful Substance-Containing Liquid, and Method for Manufacturing Useful Substance>

The cell suspension obtained using the method for manufacturing a cell suspension described above may contain a microcarrier, adherent cells that are adhered to the microcarrier, and one or more useful substances. These useful substances may be substances secreted by the adherent cells, including extracellular vesicles such as exosomes, microvesicles and apoptotic bodies, and functional proteins such as cytokines, hormones and antibodies.

In one embodiment of the present disclosure, a method for manufacturing a useful substance-containing liquid includes providing a cell suspension obtained using one of the embodiments described above, and obtaining a useful substance-containing liquid from the cell suspension. The method for manufacturing a useful substance-containing liquid may also include other optional steps.

An example of an optional step in the method for manufacturing a useful substance-containing liquid is an additional culture step for collecting the useful substance. **In** the additional culture step, a collection medium may be used that enables efficient harvest of the useful substance produced inside the cells as a result of the above method for manufacturing a cell suspension. Examples of the collection medium include media such as FBS that do not contain additives containing exosomes and are capable of maintaining cell growth, and these media may be selected appropriately in accordance with factors such as the type of useful substance and the type of cell. For example, in the case where the useful substance contains an exosome, a medium such as DMEM/F12 containing FGF-2, insulin, transferrin and selenium may be used.

By separating the microcarrier and adherent cells in the cell suspension from the useful substance and collecting the liquid containing the useful substance, a useful substance-containing liquid can be obtained. Conventional separation methods such as supernatant collection and centrifugal separation may be used for the separation. For example, the useful substance-containing liquid can be obtained by removing the microcarrier and the adherent cells from the cell suspension. The useful substance-containing liquid may not contain any substantial microcarrier or adherent cells. In this description, the expression that "the useful substance-containing liquid may not contain any substantial microcarrier or adherent cells" means that, for example, 100 mL of the useful substance-containing liquid contains no more than two cells and no more than one microcarrier having an average particle size of 50 µm or greater.

In one embodiment of the present disclosure, a method for manufacturing a useful substance includes providing a cell suspension obtained using the embodiment described above, or providing a useful substance-containing liquid obtained using the embodiment described above, and obtaining the useful substance from the cell suspension or the useful substance-containing liquid. The method for manufacturing a useful substance may also include other optional steps.

The useful substance can be obtained by separating the useful substance from the other components in the useful substance-containing liquid, and harvesting the useful substance. Conventional separation methods such as supernatant removal and centrifugal separation may be used for the separation. For example, the useful substance can be obtained by isolating the useful substance from the cell suspension.

In one embodiment, the useful substance contains exosomes. Exosomes are vesicles which contain a lipid bilayer. The diameters of the exosomes are, for example, within a range from 50 to 1,000 nm, from 50 to 300 nm, or from 50 to 200 nm. Exosomes include a variety of physiologically active substances such as proteins, nucleic acids, saccharides and lipids, and are therefore expected to be of great use in treatment methods and diagnostic methods for diseases, and in drugs and cosmetics and the like.

In those cases where the adherent cells exist in a cell suspension, the exosomes are secreted from the adherent cells into the cell suspension. There are no particular limitations on the exosomes derived from the adherent cells, provided they can be obtained from the adherent cells described above. Examples include the exosomes disclosed in WO 2009/105044.

The useful substance-containing liquid may be subjected to filtration, concentration, or a combination of filtration and concentration. For example, the useful substance-containing liquid may be filtered using a membrane having a specific size or molecular weight cutoff value. Alternatively, the useful substance-containing liquid may be filtered or concentrated using tangential flow filtration or ultrafiltration.

The useful substance in a useful substance-containing liquid may be isolated from the useful substance-containing liquid prior to use. In such a case, the useful substance in the useful substance-containing liquid may be isolated from the useful substance-containing liquid, for example by subjecting the useful substance-containing liquid to a conventional treatment such as spray drying or freeze drying.

In those cases where the useful substance-containing liquid contains exosomes, the exosomes and the other components in the exosome-containing liquid can be separated based on the properties of the exosomes. The exosomes can be isolated from the exosome-containing liquid based on the properties of the exosomes.

For example, the exosomes can be isolated on the basis of molecular weight, size, shape, composition or physical activity. Specific examples include precipitate isolation by ultracentrifugation, fraction isolation by density gradient ultracentrifugation, isolation using size exclusion chromatography, isolation using ion exchange chromatography (for example, CIMmultus^{™} EV (manufactured by BIA Separations Ltd.)), isolation by capture using proteins (for example, MagCapture^{™} Exosome Isolation Kit PS (manufactured by FUJIFILM Wako Pure Chemical Corporation)), isolation by capture using antibodies, and precipitate isolation using a polymer such as polyethylene glycol. These methods may be used individually, or a plurality of methods may be combined.

Exosome properties can be used, in the method for manufacturing an exosome-containing liquid or the method for manufacturing an exosome, for tracking the exosome activity. For example, the exosome activity can be confirmed by static light scattering, dynamic light scattering, or using a UV-vis detector, a fluorescence detector or a differential refractive index detector.

### <Examples of Embodiments>

Examples of embodiments of the present invention are presented below. Embodiments of the present invention are not limited to the following embodiments.
[1] A method for manufacturing a cell suspension, the method including (A), (B) and (C) described below:
   (A) culturing adherent cells in a cell suspension containing the adherent cells, a microcarrier and a medium, and having a volume of at least 0.3 L,
   (B) culturing the adherent cells in a cell suspension containing the adherent cells obtained through (A), a microcarrier and a medium, and having a volume of at least 5 L, and
   (C) culturing the adherent cells in a cell suspension containing the adherent cells obtained through (B), a microcarrier and a medium, and having a volume of at least 10 L,

   wherein a volume of the cell suspension in (B) is greater than a volume of the cell suspension in (A), and a volume of the cell suspension in (C) is greater than a volume of the cell suspension in (B), and
      at least one selected from the group consisting of (A), (B) and (C) includes conducting intermittent agitation of the cell suspension,
   the intermittent agitation refers to alternately conducting agitation for a prescribed period and then not conducting agitation for a prescribed period,
   the time for which agitation is conducted is within a range from 0.5 to 60 minutes, and the time for which agitation is not conducted is within a range from 0.1 to 10,

   (A) includes obtaining a cell suspension containing the adherent cells, a fresh microcarrier and a medium and having a volume of at least 0.3 L, and culturing the adherent cells,
   (B) includes obtaining a cell suspension containing the adherent cells obtained through (A), a fresh microcarrier and a medium and having a volume of at least 5 L, and culturing the adherent cells, and
   (C) includes obtaining a cell suspension containing the adherent cells obtained through (B), a fresh microcarrier and a medium and having a volume of at least 10 L, and culturing the adherent cells.
[2] The method for manufacturing a cell suspension according to [1], wherein the concentration of the adherent cells in the cell suspension (A1) obtained by (A) prior to culturing is within a range from 1×10³ to 2×10⁵ cells/mL,
   the concentration of the adherent cells in the cell suspension (B1) obtained by (B) prior to culturing is within a range from 1×10³ to 2×10⁵ cells/mL, and
   the concentration of the adherent cells in the cell suspension (C1) obtained by (C) prior to culturing is within a range from 1×10³ to 2×10⁵ cells/mL.
[3] The method for manufacturing a cell suspension according to [1] or [2], wherein the adherent cells obtained through (A) and the adherent cells obtained through (B) include a population of cells that are adhered to the microcarrier.
[4] The method for manufacturing a cell suspension according to [2] or [3], wherein
   (B) includes mixing at least the adherent cells obtained through (A), a fresh microcarrier and a fresh medium to obtain a cell suspension, and
   (C) includes mixing at least the adherent cells obtained through (B), a fresh microcarrier and a fresh medium to obtain a cell suspension.
[6] The method for manufacturing a cell suspension according to any one of [1] to [5], wherein the volume of the cell suspension in (C) is 30 L or greater.
[7] The method for manufacturing a cell suspension according to any one of [1] to [6], wherein at least one selected from the group consisting of (A), (B) and (C) includes conducting intermittent agitation of the cell suspension, or
   the method for manufacturing a cell suspension according to any one of [1] to [6], wherein at least one selected from the group consisting of (A), (B) and (C) includes conducting intermittent agitation of the cell suspension, and conducting continuous agitation of the cell suspension obtained through intermittent agitation.
[8] A method for manufacturing a cell suspension, the method including obtaining a cell suspension containing adherent cells, a microcarrier and a medium and conducting intermittent agitation of the cell suspension, and conducting continuous agitation of the cell suspension obtained through intermittent agitation. The cell suspension containing the adherent cells, the microcarrier and the medium may be the cell suspension in at least one selected from the group consisting of (A), (B) and (C) described above.
[9] A method for manufacturing adherent cells, the method including:
   providing a cell suspension obtained using the method according to any one of [1] to [8], and
   obtaining adherent cells from the cell suspension.
[10] A method for manufacturing a useful substance-containing liquid, the method including:
   providing a cell suspension obtained using the method according to any one of [1] to [8], and
   obtaining a useful substance-containing liquid from the cell suspension.
[11] A method for manufacturing a useful substance, the method including:
   providing a cell suspension obtained using the method according to any one of [1] to [8], or providing a useful substance-containing liquid obtained using the method according to [10], and
   obtaining a useful substance from the cell suspension or the useful substance-containing liquid.
[12] The method according to [10] or [11], wherein the useful substance contains at least one selected from the group consisting of an extracellular vesicle and a functional protein.

### EXAMPLES

Embodiments of the present invention are described below in further detail using a series of examples. However, embodiments of the present invention are not limited to the following examples.

### [Example 1 Manufacturing of Cell Suspension and Adherent Cells]

### <Plate Culture of hMSC>

Human bone marrow-derived mesenchymal stem cells at passage 2 (hMSC) procured from Lonza, Inc. were prepared. Alpha-Modified Eagle's Minimum Essential Medium containing nucleosides and 10% fetal bovine serum (FBS) (manufactured by Biological Industries Ltd.) (MEM alpha, nucleosides (manufactured by Gibco Inc.)) was used as the medium. The hMSCs were seeded at a density of 3,000 cells/cm² in a tissue culture flask, and cultured for 7 days in a 37°C incubator containing 5% by volume of CO₂. Using an enzyme solution (TrypLE Select, manufactured by Thermo Fisher Scientific Inc.), the cells were detached from the flask to obtain hMSCs at passage 3. The hMSCs at passage 3 were seeded at a density of 3,000 cells/cm² in a multilayer cell culture vessel (10-layer Nunc Easy Fill Cell Factory, manufactured by Thermo Fisher Scientific Inc.), and after culturing for 7 days, an enzyme treatment was used to obtain cells at passage 4. The thus obtained passage 4 hMSCs were cryopreserved in liquid nitrogen.

### <Large-Scale Culture of hMSCs (Manufacturing of Cell Suspension)>

Steps (A), (B) and (C) described below were conducted to sequentially increase the scale of the culturing, thus enabling large-scale culture of the hMSCs. FIG. 1 illustrates an outline of the method for manufacturing a cell suspension that includes steps (A), (B) and (C). The lower portions of the figure for steps (B) and (C) represent adherent cells migration between microcarriers, so-called bead-to-based cell transfer.

In the examples, unused microcarrier was used as the fresh microcarrier, namely, microcarrier for which 100% of the surface area of the cell adhesive region has no adherent cells adhered thereto. The cell adhesive region (area/mass) for Cytodex 1 is 4,400 cm²/g.

### [Step (A)]

Two disposable 2 L bioreactors (UniVessel (R) SU, manufactured by Sartorius Stedim Biotech AG) were used for culturing the hMSCs. One liter of the same medium as that described above was placed in each bioreactor, and a controller (BIOSTAT (R) B, manufactured by Sartorius Stedim Biotech AG) was used to control the temperature (37°C), the pH (7.4) and the dissolved oxygen concentration (DO) (100%) for a period of 4 hours. The above cryopreserved hMSCs were thawed in a 37°C water bath and washed. Each bioreactor was inoculated with 2.27 g of fresh microcarrier (Cytodex 1, manufactured by GE Healthcare Bio-Sciences Corporation) and 3×10⁷ of the hMSCs at passage 4, thus obtaining a cell suspension. The thus obtained cell suspension was subjected to intermittent agitation. The conditions for the intermittent agitation involved conducting 6 cycles, wherein one cycle was composed of conducting agitation for 5 minutes and subsequently conducting no agitation (leaving the cell suspension to stand) for 25 minutes. Subsequently, the cell suspension was left to stand (with no agitation) overnight. One liter of fresh medium was then added to each bioreactor to increase the volume of the cell suspension to 2 L. Subsequently, from day 1 through until day 8 of culture, the agitation speed was increased form 70 rpm to 85 rpm, and continuous agitation was conducted. On day 8 of the culture, 50% by volume of the medium was replaced, and culturing was continued until day 9.

In step (A), the cell suspension having a volume of 1 L was subjected to intermittent agitation for 3 hours and left to stand overnight (15 hours), and then the cell suspension having a volume of 2 L was subjected to continuous agitation for 7 days.

### [Step (B)]

A 50 L culture bag (Flexsafe STR, manufactured by Sartorius Stedim Biotech AG) was set into a bag holder, and 15 L of the same medium as that described above was added to the culture bag. A control tower (BIOSTAT (R) STR, manufactured by Sartorius Stedim Biotech AG) was used to control the temperature, the pH and the dissolved oxygen concentration (DO) of the medium overnight. Subsequently, 1 L of a suspension containing 18.2 g of fresh microcarrier dispersed in the medium, and 4 L of the day 9 cell suspension obtained above in step (A) were added to the 50 L culture bag, thus obtaining a cell suspension having a volume of 20 L. The thus obtained cell suspension was subjected to intermittent agitation for 25 hours. The conditions for the intermittent agitation involved conducting 12 cycles, wherein one cycle was composed of conducting agitation for 5 minutes and subsequently conducting no agitation (leaving the cell suspension to stand) for 2 hours. Subsequently, the cell suspension was subjected to continuous agitation for 4 days.

In step (B), the cell suspension having a volume of 20 L was subjected to intermittent agitation for 25 hours, and then following the intermittent agitation, was subjected to continuous agitation for 4 days.

### [Step (C)]

Subsequently, on day 13, 1 L of a cell suspension containing 34.1 g of fresh microcarrier dispersed in the medium and 29 L of warmed fresh medium were added to the culture bag. The volume of the cell suspension increased to 50 L. In a similar manner to step (B), the cell suspension was subjected to intermittent agitation for 25 hours. The cell suspension was then subjected to continuous agitation for 7 days, and on day 20, 50% by volume of the medium was replaced. Following the medium replacement, the cell suspension was subjected to continuous agitation for 7 days.

In step (C), the cell suspension having a volume of 50 L was subjected to intermittent agitation for 25 hours, and then following the intermittent agitation, was subjected to continuous agitation for a total of 14 days.

### [Evaluations of Cell Density, Viability, and Total Cell Number]

In steps (A) to (C), samples were harvested each day from the cell suspension undergoing cell culture. Using the harvested samples, evaluations were conducted of the cell density (cells/mL), the viability (%), and the total cell number (cells). The evaluation results are shown in Table 1. In the table fields for step (A), results for the cell suspension having a total volume of 4 L are shown. The cell density result for day 0 was also calculated assuming a total volume of 4 L.

**[Table 1]**

| Step | Culture duration (days) | Cell density [cells/ml] | Viability [%] | Total cell number [cells] |
|---|---|---|---|---|
| (A) | 0 | 1.50E+04 | 99.0 | 6.00E+07 |
| | 1 | 2.11E+04 | 98.6 | 8.44E+07 |
| | 2 | 1.99E+04 | 94.5 | 7.96E+07 |
| | 3 | 1.96E+04 | 84.8 | 7.85E+07 |
| | 5 | 3.15E+04 | 83.7 | 1.26E+08 |
| | 6 | 3.63E+04 | 89.2 | 1.45E+08 |
| | 7 | 6.12E+04 | 93.9 | 2.45E+08 |
| | 8 | 7.43E+04 | 96.1 | 2.97E+08 |
| | 9 | 1.50E+05 | 94.1 | 4.43E+08 |
| (B) | 10 | 3.31E+04 | 98.8 | 6.62E+08 |
| | 12 | 6.57E+04 | 98.8 | 1.31E+09 |
| | 13 | 1.06E+05 | 98.0 | 2.12E+09 |
| (C) | 14 | 5.16E+04 | 98.5 | 2.58E+09 |
| | 15 | 5.38E+04 | 85.7 | 2.69E+09 |
| | 16 | 5.59E+04 | 90.5 | 2.80E+09 |
| | 18 | 8.31E+04 | 91.4 | 4.16E+09 |
| | 19 | 1.11E+05 | 92.5 | 5.54E+09 |
| | 20 | 1.31E+05 | 96.5 | 6.57E+09 |
| | 21 | 1.70E+05 | 97.0 | 8.49E+09 |
| | 22 | 2.16E+05 | 98.2 | 1.08E+10 |
| | 23 | 2.44E+05 | 97.7 | 1.22E+10 |
| | 26 | 2.89E+05 | 98.1 | 1.45E+10 |
| | 27 | 2.91E+05 | 98.1 | 1.46E+10 |

| | | | | |
|---|---|---|---|---|
| In the table, [aE+b] means [a×10^{b}] | | | | |

In each step, the cell density increased over time, and reached 2.9×10⁵ cells/mL on day 27. Although cell viability dropped temporarily following the addition of fresh microcarrier to the cell suspension, cell viability was maintained at 83.7% or higher throughout the entire culture period, and reached 98.1% on day 27. The total cell number increased from 6.00×10⁷ cells to 1.46×10¹⁰ cells, with a 243-fold increase in the total cell number during the 27-day culture.

### [Observation by Fluorescence Microscopy]

Using the samples harvested on days 0, 9, 10, 13, 14 and 27 in Table 1, the cells adhered to the microcarriers were observed using a fluorescence microscope (manufactured by Keyence Corporation). In the samples harvested on days 0, 10 and 14, the formation of microcarrier aggregates was suppressed, and almost all microcarriers were confirmed as having adhered cells. In the samples harvested on days 9, 13 and 27, the cells were confirmed as having proliferated to confluence on the microcarrier surfaces.

### <Harvest of hMSCs (Manufacturing of Adherent Cells)>

A sample for harvesting the cells was harvested from the cell suspension under agitation on day 27 and transferred to a culture vessel. The sample was left to stand, and once the microcarriers had settled, the supernatant was removed, and the microcarriers were washed twice using phosphate buffered saline (PBS) containing no magnesium or calcium ions. An enzyme solution (TrypLE Select, manufactured by Thermo Fisher Scientific Inc.) was added to the microcarriers, and the culture vessel was shaken for 12 minutes using a small constant-temperature incubator shaker (BioShaker, manufactured by Taitec Corporation). Detachment of the cells from the microcarriers was confirmed under a phase contrast microscope (manufactured by Olympus Corporation). A medium was added to the cell suspension containing the detached cells and the microcarriers, and the cells were separated from the microcarriers and harvested using a mesh filter (Falcon^{™} mesh, hole size: 50 µm, manufactured by Corning Inc.).

The harvested cells were confirmed as having proliferation ability, expressing surface markers (CD73, CD90 and CD105), and maintaining tri-lineage differentiation potential into adipocytes, osteocytes and chondrocytes.

### [Example 2 Manufacturing of Useful Substance-Containing Liquid]

### <Manufacturing of Exosome-Containing Liquid (Useful Substance-Containing Liquid)>

With the exceptions of using adipose-derived mesenchymal stem cells (manufactured by Lonza, Inc.) as the cells for culturing, and in step (C), conducting intermittent agitation of the cell suspension with a volume of 50 L for 25 hours, and then conducting a total of 12 days of continuous agitation following the intermittent agitation, the same method as that described for steps (A), (B) and (C) in Example 1 is used to culture the adipose-derived mesenchymal stem cells to obtain a cell suspension with a volume of 50 L containing the adipose-derived stem cells.

Step (C) is halted on day 12 from the start of step (C), the cultured cells are harvested, and 50 L of PBS is then added to wash the cells. Following removal of the PBS, 50 L of DMEM/F12 (manufactured by Gibco Inc.) containing 10 ng/mL of FGF-2 (manufactured by BioVision Inc.) and 1× ITS (insulin·transferrin·selenium, manufactured by In Vitria Inc.) is added, and the resulting mixture is cultured for 48 hours under stirring at an appropriate revolution rate, thus obtaining a cell suspension.

Using the 0.65 µm module of a tangential flow filtration device (manufactured by Repligen Corporation), floating adherent cells, microcarriers and cell debris are removed from the cell suspension using to obtain an exosome-containing liquid.

### <Manufacturing of Exosome Concentrate (Useful Substance-Containing Liquid)>

Using the 0.2 µm module of a tangential flow filtration device (manufactured by Repligen Corporation), particles having a particle size of 200 nm or greater are removed from 50 L of the exosome-containing liquid obtained above. Subsequently, using the module with a molecular weight cut-off (MWCO) of 500 kDa, the liquid is concentrated down to 50 mL, thus obtaining an exosome concentrate.

### <Manufacturing 1 of Exosome Purified Liquid (Useful Substance-Containing Liquid)>

Using an ultracentrifuge XE-90 and a Swing Rotor SW 41 Ti (both manufactured by Beckman Coulter Inc.), 50 mL of the exosome concentrate obtained above is subjected to centrifugal separation at 35,000 rpm for 70 minutes to cause settling of the exosomes. The supernatant is removed, 10 mL of PBS is added to the settled exosomes, and following mixing with a vortex mixer, the exosomes are again caused to settle at 35,000 rpm for 70 minutes. Following removal of the supernatant, 30 µL of PBS is added, and pipetting is used to collect the exosomes.

### <Manufacturing 2 of Exosome Purified Liquid (Useful Substance-Containing Liquid)>

Using a MagCapture^{™} exosome isolation kit PS (manufactured by FUJIFILM Wako Pure Chemical Corporation), the exosomes in 50 mL of the exosome concentrate obtained above are purified in accordance with the kit protocol.

### <Manufacturing 3 of Exosome Purified Liquid (Useful Substance-Containing Liquid)>

Fifty mL of the exosome concentrate obtained above is purified using a chromatography system FPLC AKTA pure 150 (manufactured by AKTA) and a 1 mL volume monolith column (manufactured by BIA Separations Ltd.). Using an aqueous solution containing 50 mM of a HEPES buffer and 20 mM of NaCl (pH 7.0) as the mobile phase, the exosomes are supported on the monolith column that has been pretreated in accordance with standard protocols, and the mobile phase is then flowed through the column for one hour to wash the column. After washing, the mobile phase is changed to an aqueous solution containing 50 mM of the HEPES buffer and 2.0 M of NaCl, and this mobile phase is passed through the column at 1 mL/minute to harvest the supported exosomes.

### <Measurement of Particle Size Distribution and Concentration of Exosomes>

The particle size distribution and concentration of the exosomes in each of the exosome purified liquids purified above are measured using a nanoparticle tracking device Zeta View (manufactured by Particle Metrix GmbH), using the EV measurement method included with the software. The measurement conditions included a sensitivity setting of 82 and a shutter setting of 100.

This enables confirmation of the particle size distribution and concentration of the exosomes, and for example, yields results of 20 to 500 nm for the particle size distribution (based on scattered light intensity), and 10¹⁰ to 10¹¹ particles/mL for the concentration.

### <Evaluation of Protein Expression of Exosomes>

Using 20 µL of each of the exosome purified liquids purified above, a DC assay (manufactured by Bio-Rad Laboratories, Inc.) is used to quantify the total protein amount of the exosomes. Sufficient exosome purified liquid is then weighed to achieve a total protein amount for the exosome purified liquid of 0.5 µg, and the sample is mixed with 4 µL of 4× SDS-PAGE Sample buffer (manufactured by Tokyo Chemical Industry Co., Ltd.). Additional distilled water is added to the thus obtained mixed liquid to make the total volume up to 16 µL, and the mixture is then heated at 37°C for 5 minutes. Subsequently, the mixed liquid is applied to a 10% polyacrylamide gel (manufactured by ATTO Corporation), and an electrophoresis device (manufactured by ATTO Corporation) is used to conduct phoretic separation of the proteins under conditions including a protein amount of 500 ng/lane, 150 V and 30 minutes. Following phoresis, the proteins in the gel are transferred to a polyvinylidene fluoride (PVDF) membrane (manufactured by ATTO Corporation) using a transfer device (manufactured by ATTO Corporation) under conditions of 100 V and 15 minutes. Following blocking of the membrane with a blocking buffer (manufactured by Nacalai Tesque, Inc.), reactions are conducted with 1 µg/mL anti-human CD9 mouse IgG, anti-human CD63 mouse IgG, and anti-human CD81 mouse IgG antibodies (each manufactured by Cosmo Bio Co., Ltd.) at 4°C across an 18-hour period. Following reaction, the membrane is washed with TBS buffer, and then reacted at room temperature for one hour with 0.2 µg/mL of mouse-HRP antibody. Following reaction, the membrane is washed with TBS buffer and visualized with ImmunoStar LD (manufactured by FUJIFILM Wako Pure Chemical Corporation), and expression of each of CD9, CD63 and CD81 is confirmed using a chemiluminescence imaging system (manufactured by FUJIFILM Wako Pure Chemical Corporation).

This procedure confirms expression of CD9, CD63 and CD81 by the exosomes. Further, by using various other antibodies, protein expression of the exosomes is able to be evaluated. For example, the expression of Hsp70, TSG101, and tubulin and the like is able to be confirmed.

The disclosure of the present application is related to the subject matter disclosed in prior Japanese Application 2020-43974 filed on March 13, 2020, the entire content of which is incorporated by reference herein. Further, the entire contents of the documents disclosed in the present description are incorporated by reference herein.

## Claims

1. A method for manufacturing a cell suspension, the method comprising (A), (B) and (C) described below:
(A) culturing adherent cells in a cell suspension containing the adherent cells, a microcarrier and a medium, and having a volume of at least 0.3 L,
(B) culturing the adherent cells in a cell suspension containing the adherent cells obtained through (A), a microcarrier and a medium, and having a volume of at least 5 L, and
(C) culturing the adherent cells in a cell suspension containing the adherent cells obtained through (B), a microcarrier and a medium, and having a volume of at least 10 L,
wherein a volume of the cell suspension in (B) is greater than a volume of the cell suspension in (A), and a volume of the cell suspension in (C) is greater than a volume of the cell suspension in (B), **characterized in that**
at least one selected from the group consisting of (A), (B) and (C) includes conducting intermittent agitation of the cell suspension,
the intermittent agitation refers to alternately conducting agitation for a prescribed period and then not conducting agitation for a prescribed period,
the time for which agitation is conducted is within a range from 0.5 to 60 minutes, and the time for which agitation is not conducted is within a range from 0.1 to 10 hours,
(A) includes obtaining a cell suspension containing the adherent cells, a fresh microcarrier and a medium and having a volume of at least 0.3 L, and culturing the adherent cells,
(B) includes obtaining a cell suspension containing the adherent cells obtained through (A), a fresh microcarrier and a medium and having a volume of at least 5 L, and culturing the adherent cells, and
(C) includes obtaining a cell suspension containing the adherent cells obtained through (B), a fresh microcarrier and a medium and having a volume of at least 10 L, and culturing the adherent cells.

2. The method for manufacturing a cell suspension according to Claim 1, wherein
the concentration of the adherent cells in the cell suspension (A 1) obtained by (A) prior to culturing is within a range from 1×10³ to 2×10⁵ cells/mL,
the concentration of the adherent cells in the cell suspension (B1) obtained by (B) prior to culturing is within a range from 1×10³ to 2×10⁵ cells/mL, and
the concentration of the adherent cells in the cell suspension (C1) obtained by (C) prior to culturing is within a range from 1×10³ to 2×10⁵ cells/mL.

3. The method for manufacturing a cell suspension according to Claim 1 or 2, wherein the adherent cells obtained through (A) and the adherent cells obtained through (B) include a population of cells that are adhered to a microcarrier.

4. The method for manufacturing a cell suspension according to Claim 2 or 3, wherein
(B) includes mixing at least the adherent cells obtained through (A), a fresh microcarrier and a fresh medium to obtain a cell suspension, and
(C) includes mixing at least the adherent cells obtained through (B), a fresh microcarrier and a fresh medium to obtain a cell suspension.

5. The method for manufacturing a cell suspension according to any one of Claim 1 to 4, wherein a volume of the cell suspension in (C) is 30 L or greater.

6. A method for manufacturing adherent cells, the method comprising:
providing a cell suspension using the method according to any one of Claims 1 to 5, and
obtaining adherent cells from the cell suspension.

7. A method for manufacturing a useful substance-containing liquid, the method comprising:
providing a cell suspension using the method according to any one of Claims 1 to 5, and
obtaining a useful substance-containing liquid from the cell suspension,
wherein the useful substance comprises at least one selected from the group consisting of an extracellular vesicle and a functional protein.

8. A method for manufacturing a useful substance, the method comprising:
providing a cell suspension using the method according to any one of Claims 1 to 5, or providing a useful substance-containing liquid using the method according to Claim 7, and
obtaining a useful substance from the cell suspension or the useful substance-containing liquid, wherein the useful substance comprises at least one selected from the group consisting of an extracellular vesicle and a functional protein.

## Patentansprüche

1. Verfahren zum Herstellen einer Zellsuspension, wobei das Verfahren die folgenden Schritte (A), (B) und (C) umfasst:
(A) Kultivieren von adhärenten Zellen in einer Zellsuspension, die die adhärenten Zellen, einen Mikroträger und ein Medium enthält und ein Volumen von mindestens 0,3 L aufweist,
(B) Kultivieren der adhärenten Zellen in einer Zellsuspension, die die adhärenten Zellen, die durch (A) erhalten wurden, einen Mikroträger und ein Medium enthält und ein Volumen von mindestens 5 L hat, und
(C) Kultivieren der adhärenten Zellen in einer Zellsuspension, die die adhärenten Zellen, die durch (B) erhalten wurden, einen Mikroträger und ein Medium enthält und ein Volumen von mindestens 10 L hat,
wobei ein Volumen der Zellsuspension in (B) größer ist als ein Volumen der Zellsuspension in (A) und ein Volumen der Zellsuspension in (C) größer ist als ein Volumen der Zellsuspension in (B), **dadurch gekennzeichnet, dass**
mindestens ein Schritt ausgewählt aus der Gruppe bestehend aus (A), (B) und (C) die Durchführung eines intermittierenden Rührens der Zellsuspension aufweist,
wobei das intermittierende Rühren sich auf das abwechselnde Durchführen des Rührens für einen vorgegebenen Zeitraum und das anschließende Nichtdurchführen des Rührens für einen vorgegebenen Zeitraum bezieht,
wobei die Zeit, für die ein Rühren durchgeführt wird, in einem Bereich von 0,5 bis 60 Minuten liegt, und die Zeit, für die kein Rühren durchgeführt wird, in einem Bereich von 0,1 bis 10 Stunden liegt,
(A) das Erhalten einer Zellsuspension, die die adhärenten Zellen, einen frischen Mikroträger und ein Medium enthält und ein Volumen von mindestens 0,3 L hat, und das Kultivieren der adhärenten Zellen beinhaltet,
(B) das Erhalten einer Zellsuspension, die die durch (A) erhaltenen adhärenten Zellen, einen frischen Mikroträger und ein Medium enthält und ein Volumen von mindestens 5 L hat, und das Kultivieren der adhärenten Zellen beinhaltet, und
(C) das Erhalten einer Zellsuspension, die die durch (B) erhaltenen adhärenten Zellen, einen frischen Mikroträger und ein Medium enthält und ein Volumen von mindestens 10 L aufweist, und das Kultivieren der adhärenten Zellen beinhaltet.

2. Verfahren zum Herstellen einer Zellsuspension gemäß Anspruch 1, wobei
die Konzentration der adhärenten Zellen in der durch (A) erhaltenen Zellsuspension (A1) vor der Kultivierung in einem Bereich von 1 × 10³ bis 2 × 10⁵ Zellen/mL liegt,
die Konzentration der adhärenten Zellen in der durch (B) erhaltenen Zellsuspension (B1) vor der Kultivierung in einem Bereich von 1 × 10³ bis 2 × 10⁵ Zellen/mL liegt und
die Konzentration der adhärenten Zellen in der Zellsuspension (C1), die durch (C) vor der Kultivierung erhalten wird, in einem Bereich von 1 × 10³ bis 2 × 10⁵ Zellen/mL liegt.

3. Verfahren zum Herstellen einer Zellsuspension gemäß Anspruch 1 oder 2, wobei die durch (A) erhaltenen adhärenten Zellen und die durch (B) erhaltenen adhärenten Zellen eine Population von Zellen beinhalten, die an einem Mikroträger adhärieren.

4. Verfahren zum Herstellen einer Zellsuspension gemäß Anspruch 2 oder 3, wobei
(B) das Mischen mindestens der durch (A) erhaltenen adhärenten Zellen, eines frischen Mikroträgers und eines frischen Mediums umfasst, um eine Zellsuspension zu erhalten, und
(C) das Mischen mindestens der durch (B) erhaltenen adhärenten Zellen, eines frischen Mikroträgers und eines frischen Mediums umfasst, um eine Zellsuspension zu erhalten.

5. Verfahren zum Herstellen einer Zellsuspension gemäß einem der Ansprüche 1 bis 4, wobei ein Volumen der Zellsuspension in (C) 30 L oder mehr beträgt.

6. Verfahren zum Herstellen adhärenter Zellen, wobei das Verfahren umfasst:
Bereitstellen einer Zellsuspension unter Verwendung des Verfahrens gemäß einem der Ansprüche 1 bis 5, und
Erhalten adhärenter Zellen aus der Zellsuspension.

7. Verfahren zur Herstellung einer nützlichen Substanz enthaltenden Flüssigkeit, wobei das Verfahren umfasst:
Bereitstellen einer Zellsuspension unter Verwendung des Verfahrens gemäß einem der Ansprüche 1 bis 5, und
Erhalten einer nützlichen Substanz enthaltenden Flüssigkeit aus der Zellsuspension,
wobei die nützliche Substanz mindestens eine Substanz umfasst, die aus der Gruppe ausgewählt ist, die aus einer extrazellulären Vesikel und einem funktionellen Protein besteht.

8. Verfahren zum Herstellen einer nützlichen Substanz, wobei das Verfahren umfasst:
Bereitstellen einer Zellsuspension unter Verwendung des Verfahrens gemäß einem der Ansprüche 1 bis 5, oder Bereitstellen einer nützlichen Substanz enthaltenden Flüssigkeit unter Verwendung des Verfahrens gemäß Anspruch 7, und
Erhalten einer nützlichen Substanz aus der Zellsuspension oder der nützlichen Substanz enthaltenden Flüssigkeit,
wobei die nützliche Substanz mindestens eine Substanz umfasst, die aus der Gruppe ausgewählt ist, die aus einer extrazellulären Vesikel und einem funktionellen Protein besteht.

## Revendications

1. Procédé de production d'une suspension cellulaire, le procédé comprenant (A), (B) et (C) décrits ci-dessous :
(A) la culture de cellules adhérentes dans une suspension cellulaire contenant les cellules adhérentes, un microsupport et un milieu, et présentant un volume d'au moins 0,3 L,
(B) la culture des cellules adhérentes dans une suspension cellulaire contenant les cellules adhérentes obtenues par (A), un microsupport et un milieu, et présentant un volume d'au moins 5 L, et
(C) la culture des cellules adhérentes dans une suspension cellulaire contenant les cellules adhérentes obtenues par (B), un microsupport et un milieu, et présentant un volume d'au moins 10 L,
dans lequel un volume de la suspension cellulaire dans (B) est supérieur à un volume de la suspension cellulaire dans (A), et un volume de la suspension cellulaire dans (C) est supérieur à un volume de la suspension cellulaire dans (B), **caractérisé en ce que**
au moins un élément sélectionné dans le groupe consistant en (A), (B) et (C) inclut la réalisation d'une agitation intermittente de la suspension cellulaire,
l'agitation intermittente fait référence au fait de procéder alternativement à l'agitation pendant une période prescrite, puis de ne pas procéder à l'agitation pendant une période prescrite,
le temps pendant lequel l'agitation est effectuée est compris dans une plage de 0,5 à 60 minutes, et le temps pendant lequel l'agitation n'est pas effectuée est compris dans une plage de 0,1 à 10 heures,
(A) inclut l'obtention d'une suspension cellulaire contenant les cellules adhérentes, un microsupport frais et un milieu et présentant un volume d'au moins 0,3 L, et la culture des cellules adhérentes,
(B) inclut l'obtention d'une suspension cellulaire contenant les cellules adhérentes obtenues par (A), un microsupport frais et un milieu et présentant un volume d'au moins 5 L, et la culture des cellules adhérentes, et
(C) inclut l'obtention d'une suspension cellulaire contenant les cellules adhérentes obtenues par (B), un microsupport frais et un milieu et présentant un volume d'au moins 10 L, et la culture des cellules adhérentes.

2. Procédé de production d'une suspension cellulaire selon la revendication 1,
la concentration des cellules adhérentes dans la suspension cellulaire (A1) obtenue par (A) avant la culture est comprise dans la plage de 1×10³ à 2×10⁵ cellules/ml,
la concentration des cellules adhérentes dans la suspension cellulaire (B1) obtenue par (B) avant la culture est comprise dans la plage de 1×10³ à 2×10⁵ cellules/ml, et
la concentration des cellules adhérentes dans la suspension cellulaire (C1) obtenue par (C) avant la culture est comprise dans la plage de 1×10³ à 2×10⁵ cellules/ml.

3. Procédé de production d'une suspension cellulaire selon la revendication 1 ou 2, dans lequel les cellules adhérentes obtenues par (A) et les cellules adhérentes obtenues par (B) incluent une population de cellules qui adhèrent à un microsupport.

4. Procédé de production d'une suspension cellulaire selon la revendication 2 ou 3, dans lequel
(B) inclut le mélange d'au moins les cellules adhérentes obtenues par (A), d'un microsupport frais et d'un milieu frais pour obtenir une suspension cellulaire, et
(C) inclut le mélange d'au moins les cellules adhérentes obtenues par (B), d'un microsupport frais et d'un milieu frais pour obtenir une suspension cellulaire.

5. Procédé de production d'une suspension cellulaire selon l'une quelconque des revendications 1 à 4, dans lequel un volume de la suspension cellulaire dans (C) est de 30 L ou plus.

6. Procédé de production de cellules adhérentes, le procédé comprenant :
la fourniture d'une suspension cellulaire en utilisant le procédé selon l'une quelconque des revendications 1 à 5,
et
l'obtention de cellules adhérentes à partir de la suspension cellulaire.

7. Procédé de production d'un liquide contenant une substance utile, le procédé comprenant :
la fourniture d'une suspension cellulaire en utilisant le procédé selon l'une quelconque des revendications 1 à 5,
et
l'obtention d'un liquide contenant une substance utile à partir de la suspension cellulaire, dans lequel la substance utile comprend au moins un élément sélectionné dans le groupe consistant en une vésicule extracellulaire et une protéine fonctionnelle.

8. Procédé de production d'une substance utile, le procédé comprenant :
la fourniture d'une suspension cellulaire en utilisant le procédé selon l'une quelconque des revendications 1 à 5, ou
la fourniture d'un liquide contenant une substance utile en utilisant le procédé selon la revendication 7, et
l'obtention d'une substance utile à partir de la suspension cellulaire ou du liquide contenant une substance utile,
dans lequel la substance utile comprend au moins un élément sélectionné dans le groupe consistant en une vésicule extracellulaire et une protéine fonctionnelle.
